# EUROPÄISCHE PATENTSCHRIFT

(11) **EP 2 044 438 B1**
(45) Veröffentlichungstag und Bekanntmachung des Hinweises auf die Patenterteilung: **01.11.2017**
(21) Anmeldenummer: 07817410.9
(22) Anmeldetag: 23.07.2007
(51) Int. Cl.: G01N 33/543

(54) **ANORDNUNG FÜR ONLINE-MESSUNGEN AN ZELLEN**
ARRANGEMENT FOR ON-LINE MEASUREMENTS ON CELLS
INSTALLATION POUR DES MESURES EN LIGNE SUR DES CELLULES

(30) Priorität: 24.07.2006 DE 102006034606
(43) Veröffentlichungstag der Anmeldung: 08.04.2009
(73) Patentinhaber: Biocer-Entwicklungs-GmbH, 95447 Bayreuth (DE)
(72) Erfinder: SEITZ, Daniel, 95463 Bindlach (DE); MAYR, Helmar, 95445 Bayreuth (DE); ZIEGLER, Günter, 95447 Bayreuth (DE); VONAU, Winfried, 09326 Geringswalde (DE); GERLACH, Frank, 04736 Waldheim (DE); HERRMANN, Sigrun, 04736 Waldheim (DE)
(74) Vertreter: Donath, Dirk
(86) Internationale Anmeldenummer: PCT/DE2007/001326
(87) Internationale Veröffentlichungsnummer: WO 2008/011876

(56) Entgegenhaltungen:
- WO-A-03/082469
- WO-A-2005/097969
- WO-A-2006/037022
- US-A- 5 614 378

## Beschreibung

Die Erfindung betrifft eine Anordnung (eine Messeinrichtung) für Online-Messungen an Zellen, insbesondere für die Messung löslicher Analyte bzw. gelöster Gase an Proben in einem Probenraum.

Die Detektion des Verhaltens von Zellkulturen kann, insbesondere hinsichtlich Stoffwechsel-physiologischer Parameter, mit verschiedensten elektro- oder biochemischen Sensoren erfolgen.

Die DE 41 15 792 offenbart bspw. eine grundsätzliche Anordnung für ein Biosensorsystem, welche für biochemische Messverfahren unter Verwendung einer membranbedeckten Miniaturelektrode geeignet ist und eine Membranspann- und Membranwechselvorrichtung besitzt. Gemäß dieser Schrift besitzt die Gegenelektrode der Miniaturelektrode die Form einer Kugelkalotte mit einer Mittelöffnung, innerhalb derer der Kopf der Messelektrode angeordnet ist.
Die Membranspann- und Membranwechselvorrichtung besteht aus einem zum Wechseln der Membran klappbaren Unterteil und einem damit verbundenen aufklappbaren Unterteil, wobei im erstgenannten Unterteil in einer eine Durchgangsbohrung erweiternde zylindrische Aussparung die Membran mittels eines Teflonringes vorgespannt und abgedichtet wird.
Über eine Mittelbohrung im Teflonring wird die Membran durch den Kopf der Miniaturelektrode nochmals gespannt und durch die Oberkante des Isolierkörpers nochmals abgedichtet.
Diese grundsätzliche Anordnung ist jedoch nicht für online-Messungen an Zellkulturen über lange Zeiträume verwendbar, da die Membrangrundsätzlich nicht im Probenraum integriert werden kann und nicht für die Kultur von Zellen ausgelegt ist. Insbesondere bei adhärenten Zellkulturen ist eine stabile, nicht bewegliche Unterlage notwendig. Bei der beschriebenen Anordnung ist eine störungsfreie Trennung vin Kulturraum und Sensoren nicht möglich.
Bei der Detektion des Verhaltens von Zellkulturen mittels elektrochemischer Sensoren ist es nämlich unvermeidbar, dass mit diesen Sensoren nur über eine begrenzte Zeit richtige Messwerten erzielt werden können.
Bekannter weise erfordert der Einsatz fast aller Chemosensoren und aller Biosensoren, für die meist Chemosensoren den sog. Grundsensor bilden, deren ständige Nachkalibrierung, weil funktionsbedingt u.a. relativ dünne Schichten bzw. Schichtsysteme verschiedenster Zusammensetzung, organische und/oder anorganische kristalline oder amorphe Membranen oder ggf. fluide Funktionselemente Bestandteile des Sensors sind. Diese unterliegen in Abhängigkeit von mehreren Parametern (Alter, Umgebungsmillieu, Druck, Temperatur, ...) einer stofflichen Veränderung, die sich unmittelbar auf das generierte Sensorsignal auswirkt.
Somit können bspw. die von den Zellen einer Zellkultur belegten Arrays in der Regel nur max. drei Tage zu Untersuchungen eingesetzt werden. D.h., die Anwendbarkeit für die Online-Messung beschränkt sich auf Prozesse, die innerhalb dieses Zeitraums entweder vollständig oder zumindest soweit abgelaufen sind, dass eine signifikante Aussage über den Verlauf des interessierenden Metabolismus erfolgen kann.

Viele der geeigneten Sensormaterialien werden nicht nur durch einen Belag mit Zellen und Proteinen in ihrer Funktion gestört, es kann auch zu Problemen mit der Biokompatibilität kommen. Insbesondere adhärente Zellen, die empfindlich auf die Beschaffenheit des Untergrundes reagieren, können so nicht in ihrem natürlichen Zustand untersucht werden.
Bei der Zellkultivierung im Allgemeinen und bei dreidimensionalen, vielschichtigen Tissue Engineering-Implantaten (bspw. gefäßlosem künstlichen Knorpel) im Besonderen, ist die Kontrolle des Verbrauchs von Nährstoffen durch den Stoffwechsel der Zellen essentiell für die erfolgreiche Kultivierung über längere Zeiträume. Hierzu ist es vorteilhaft, zumindest die physiologisch wichtigen Parameter Sauerstoff, Glucose und pH-Wert in den untersten Zellschichten zu messen.
Bisher wurden diese Parameter unter Zerstörung der Proben oder indirekt durch die Messung des Kulturmediums bestimmt. Neuerdings sind floureszenzbasierte Systeme mit in Silikon eingebetteten Flourophoren verfügbar, auf denen adhärente Zellen allerdings nicht wachsen. Der Einsatz o.g. bekannter Sensor-Array-Systeme scheidet wegen der Unmöglichkeit der Nachkalibrierung während des Kultivierungsprozesses aus.

Somit ist eine der größten Schwierigkeiten beim Einsatz elektrochemischer Sensoren die Interaktion der Sensoroberfläche mit der Probe. Dabei können Substanzen, wie bspw. organische Moleküle oder Proteine, mit dem Sensor interagieren und dadurch die Sensitivität des Sensors herabsetzen. Es können zudem Probleme bei der Messung an lebenden Systemen entstehen, die negativ durch die Sensoroberfläche beeinflusst werden können, z.B. bei Silber/Silberchlorid-Referenzelektroden.

Aus dem Stand der Technik sind fluoreszenzbasierte Systeme bekannt, die zwar weitestgehend bioinert sind, und damit prinzipiell für die Messung an Suspensionszellkulturen geeignet, jedoch können, wenn überhaupt, nur wenige adhärente Zelltypen auf diesem System wachsen. Ein solches System ist bspw. in DE 199 03 506, DE 100 03 673 und WO 03/036293 offenbart.
Ein weiterer Nachteil solcher fluoreszenzbasierter Systeme ist die Limitierung auf die wenigen, z.Z zur Verfügung stehenden Fluorophoren-Systeme.

Sensorsysteme, die den direkten Bewuchs mit adhärenten Zellen ermöglichen, werden u.a. in DE 197 53 598, DE 196 46 505, DE 44 17 079 und DE 196 46 505 offenbart.
Bei diesen Systemen wird eine Kompatibilität zum Zellbewuchs dadurch erreicht, dass mit Silizium-basierten Sensoren gearbeitet wird, die in Dünnschichttechnik hergestellt werden. Bei dieser Technologie, die ihre eigenen Anwendungsgebiete hat, z.B. die Messung an einzelnen Zellen, treten für die routinemäßige Anwendung im der Zell- und Gewebekultur einige Probleme auf. Insbesondere steht mit dem Silizium nur ein spezielles Material für die Adhäsion der Zellen zur Verfügung. Der Silizium-Waver, der zudem mit weiteren metallischen Materialien in der CMOS-Technik bestückt wird, bildet eine sehr spezielle Unterlage für die Adhäsion von Zellen. Da gerade die Interaktion der Gewebezellen mit der Unterlage eine wichtige Rolle in der Zellentwicklung spielt, wäre ein neutralerer Untergrund, v.a. ohne den Einfluss von elektrischen Strömen (bei amperometrischen Sensoren) und diversen Metallionen von großer Bedeutung, um mehr als nur einige spezielle Fragestellungen untersuchen zu können.

Wenn die Detektion des Verhaltens von Zellkulturen online vermittels elektrochemischer Sensoren erfolgt, ist es unvermeidbar, dass eine Nachkalibrierung bei direktem Bewuchs der Sensoren mit Zellen erfolgen müsste. Dies ist jedoch derzeit nicht möglich. Zudem sollen bei der online-Messung gerade Manipulation an der Zell- oder Gewebekultur vermieden werden.
In der Regel können die von den Zellen belegten Arrays nur max. drei Tage eingesetzt werden. D.h., die Anwendbarkeit für die Online-Messung beschränkt sich auf Prozesse, die innerhalb dieses Zeitraums entweder vollständig oder zumindest soweit abgelaufen sind, dass eine signifikante Aussage über den Verlauf des interessierenden Metabolismus erfolgen kann.

Eine weitere Problematik ergibt sich, wenn nicht punktuelle, lokale Werte einzelner Zellen erfasst, sondern integrative Gesamtdaten gemessen werden sollen. Die nebeneinander liegenden Sensoren messen jeweils unter einer anderen Zellgruppe, wobei die gegebene Variabilität bei biologischen Systemen die Korrelation so gemessener Parameter erschwert.

Schließlich ist die Herstellung von Sensoren in Dünnschichttechnik mit einem gewissen Aufwand verbunden und nur bei einem größeren Volumen wirtschaftlich sinnvoll.

Die Dickschichttechnik, die mehr Flexibilität bietet und weitaus wirtschaftlicher zu realisieren ist als die Dünnschichttechnik, führt jedoch bei den bereitgestellten Sensoren zu den selben Nachteilen, wie sie die Dünnschttechnik hervorruft.

Eine weitere Problematik ergibt sich aus der Notwendigkeit zur Sterilisation aller Teile, die mit dem Probenraum Kontakt haben. Hierbei ist ein Unterschied zu machen zwischen der einfachen Desinfizierung, die für kurze Messphasen ausreichend sein mag, und der Sterilisation, die z.B. Enzymen in Biosensoren schaden kann. Die gängigste Sterilisationsmethode im Laborbereich ist die Hochdruck-Dampfsterilisation (im Autoklav).

Membranen werden vielfach in Verbindung mit Sensoren verwendet. Bisher wurden dabei jedoch die Membranen direkt mit dem Sensor verbunden (siehe hierzu u.a. WO 87/05624, DE 697 29 185 T1 und DE 199 24 856 A1, wobei bei diesen Sensoren meist die funktionelle Selektivität im Vordergrund steht, oder mit Membran eine Enzymtragende Schicht bezeichnet wird.)
Nachteilig bei dieser technischen Lösung ist vor allem, dass eine multiparametrische Analyse der gleichen Probe und die für online-Messungen über längere Zeiträume erforderliche Nachkalibrierung nicht möglich sind.

In DE 694 11 732 T1 und EP 0289269 besteht die Membranfunktion speziell im Ausschluss von Erythrozyten bei der Blutanalyse.

In WO 199 6032 64 A1 werden die Zellen zwar auf einer Membran kultiviert, die Messung der physiologischen Parameter erfolgt jedoch durch eine im kultivierten Gewebe liegenden Sonde, die damit die Kultivierung beeinflusst, bzw. die Bedingungen ändert.

Die Schrift von D. Schepers, G. Schulze und W. Frenzel ["Spectrophotometric flow-through gas sensor for the determination of atmospheric nitrogen dioxide" (Analytika Chimica Acta 308 (1995) 109-114] offenbart eine Mikrofluidmesszelle, insbesondere für Fotometer oder Spektrometer, die vorzugsweise schmalbandig arbeiten, beinhaltend miteinander flächig verbundene Wafer, in die Mikrokanäle derart eingebracht sind, dass in zumindest einem Bereich zwei Mikrokanalabschnitte parallel zueinander verlaufend angeordnet sind und diese durch eine, entsprechend einer zu analysierenden Substanz vorgebbar ausgewählten selektiven Membran voneinander räumlich getrennt sind, so dass eine Extraktionsstrecke gebildet ist, wobei in einem ersten Wafer wenigstens genannter erster Mikrokanalabschnitt sowie dessen Kanalenden erfassende Zu- und Abströmöffnungen oder Zu- und Abströmkanäle für die Durchleitung eines Analyten eingebracht sind, in einem zweiten Wafer wenigstens genannter zweiter Mikrokanalabschnitt sowie dessen Kanalenden erfassende Zu- und Abströmöffnungen oder Zu- und Abströmkanäle für die Durchleitung eines Extraktionsmittels (E) eingebracht sind, und wenigstens ein Wafer für einen zum Einsatz gelangenden Messlichtstrahl durchlässig oder mit einem dies gewährleistenden Fensterbereich versehen ist, wobei der Ein- und Austritt des Messlichtstrahls durch den als Extraktionskanal dienenden zweiten Mikrokanalabschnitt derart festgelegt ist, dass, in Abhängigkeit von der eingesetzten Strahlungsquelle (L), eine möglichst lange optische Messstrecke realisiert ist.
Eine Online-Messung verschiedener physiologischer Parameter von Zellen einer Zellkultur, insbesondere für die Messung löslicher Analyte bzw. gelöster Gase, ist mit der zuvor stehend genannten Mikrofluidmesszelle nicht möglich, da keine spezifischen Sensoren vorgesehen sind, um die Online-Messung löslicher Analyte bzw. gelöster Gase zu realisieren.

DE 198 60 547 A1 offenbart Affinitätssensor für den Nachweis spezifischer Bindungsereignisse bestehend aus einem Trägersubstrat, das mit wenigstens zwei zueinander in einem äquidistanten Abstand angeordneten Elektroden versehen ist, die beidseitig einen Bereich erfassen, wobei zumindest dieser Bereich für die Aufnahme immobilisierter spezifischer Bindungspartner vorgesehen ist, die befähigt sind, komplementär zugehörige Bindungspartner direkt oder über weitere spezifische Bindemoleküle zu koppeln und der Bereich mit einer Mindestbreite so festgelegt ist, dass zumindest ein komplementär zugehöriger Bindungspartner, der mit einem elektrisch leitfähigem Partikel versehen ist, in genannten Bereich derart aufnehmbar ist, dass zwischem dem Partikel und den Elektroden jeweils die Möglichkeit zur Ausbildung eines Tunnelkontaktübergangs gewährleistet ist.
Eine Online-Messung an Zellen, insbesondere für die Messung löslicher Analyte bzw. gelöster Gase, ist mit dem zuvor stehend genannten Affinitätssensor nicht möglich, da nur spezifische Kopplungen von Bindemolekülen elektrisch detektiert werden können.

Die WO 03/082469 offenbart eine Vorrichtung und ein Verfahren zur Durchführung und In-Situ-Überwachung von chemischen und/oder biologischen Reaktionen.
Die Vorrichtung umfasst dabei ein Reaktionsgefäß (Zellkulturraum) mit einer Aussparung (Öffnung), in der eine Überwachungseinheit (Sensoreinheit) angeordnet ist, die wiederholt an das Reaktionsgefäß (den Zellkulturraum) angekoppelt bzw. entfernt werden kann.
Diese Überwachungseinheit (Sensoreinheit) weist an den Bereichen, die im Innern des Reaktionsgefäßes (Zellkulturraumes) angeordnet sind, eine permeable Membran auf.
Die Überwachungseinheit (Sensoreinheit) umfasst weiterhin einen Bereich, der im Innern des Reaktionsgefäßes (Zellkulturraumes) angeordnet ist, der die kontinuierliche selektive In-Situ-Erkennung eines Reaktionsteilnehmers während einer chemischen und/oder biologischen Reaktion ermöglicht und weitere Mittel zur kontinuierlichen Umwandlung der selektiven Erkennung durch Erkennungsmittel in eine Messgröße aufweist, wobei der Bereich und die Umwandlungsmittel als eine einzige Schicht (Sensorschicht) ausgebildet sind.

Die Aufgabe der vorliegenden Erfindung liegt somit darin, eine Anordnung für die Online-Messungen zur Aufklärung und Überwachung des Stoffwechsels lebender Zellen, insbesondere die Online-Messung löslicher Analyte oder gelöster Gase, anzugeben, die die zuvor stehend genannten Nachteile des Standes der Technik vermeidet, insbesondere die Nachkalibrierung während des Kultivierungsprozesses ermöglicht und Manipulationen an der Zell- oder Gewebekultur vermeidet, und dabei in ein mikrofluidisches System integrierbar ist.

Die Erfindung beinhaltet ein Sensorsystem, in dem verschiedene Sensortypen, in einer speziellen Ausführung planare Sensoren, miniaturisiert nebeneinander auf kleinstem Raum angeordnet werden. Bei der Ausführung der Sensoren ist darauf zu achten, dass keine gegenseitige Beeinflussung stattfindet. Die unmittelbar mit den Sensoren in Kontakt stehende primäre Ableitelektronik muss so ausgeführt werden, dass eine galvanische Trennung der Kontakte gewährleistet ist. Hergestellt werden kann dieses Sensorarray bspw. durch Siebdrucktechnik.
Die Erfindung beinhaltet zudem eine Membran, die optimalen Analyttransport zulässt, jedoch eine Konvektion der Medien durch die Membran hindurch weitestgehend verhindert. Die Poren müssen so eingestellt sein, dass bei optimaler Diffusion der zu messenden Analyte solche Stoffe zurückgehalten werden, die schädigend oder störend für die Sensoren sein könnten, wie etwa Proteine, feine Partikel etc. Die Membran ist biokompatibel oder bioinert, um eine nichtdestruktive Messung an Zellen oder Geweben zuzulassen. Diese können sich in Suspension befinden, oder adhärent auf der Membran wachsen. Mögliche Membranmaterialien sind Polymere, Polymerblends und Copolymere, Keramiken, Metalle und Legierungen, Glas sowie Verbundwerkstoffe.
Besonders vorteilhaft ist die Membran aus Hydroxylapatit, so dass speziell Knochen/Knorpelkulturen adhärent auf dem Material gezüchtet werden. Im Falle der Kultur von Zellen ist ein besonderer Vorteil der erfindungsgemäßen Anordnung, dass physiologische Parameter von Zell-/ Gewebekulturen kontinuierlich über einen Zeitraum von mehreren Wochen gemessen werden können. Zudem ist es vermittels dieser Anordnung möglich, wichtige biologische Parameter unterhalb der adhärenten Zell-/ Gewebekultur, also bei den am wenigsten mit Nährstoffen versorgten Zelllagen, aufzeichnen zu können.
Die nanoporöse Ausführung der Membran bewirkt die Trennung der beiden Systeme (Kulturmedium und Transfermedium) und hält die Zell-/ Gewebekultur steril.

Ein Weg zur Herstellung dieser Membranen basiert auf der Verwendung klassierter keramischer Pulver, so dass eine sehr eng verteilte Porenradienverteilung bei der Herstellung der Membran erreicht wird, die sich positiv auf die Membranfunktion (bspw. Selektivität bzgl. des Analyttransports) auswirkt.
Die Erfindung beinhaltet ein Kopplungssystem, mit dem Messeinrichtung und Probenraum voneinander getrennt sind. Dies ermöglicht
- Nachkalibrieren durch Abkoppeln und Koppeln von Eichlösungen,
- Austausch der Sensoren zur Erweiterung der messbaren Parameter
- Austausch der Probenräume zur Reihenmessung von mehreren Proben, z.B. in einem automatisierten Gerät, mit dem mehrere Membran/Probenraumeinheiten in Serie gemessen werden können.

Das Kopplungssystem ist als Bajonettverschluss ausgeführt. Gemäß der Erfindung ist der Raum (Spalt), welcher mit einem Transfermedium befüllbar ist, mit fluidischen Systemen koppelbar, so dass das Transfermedium (definierte Messflüssigkeit) in den Raum (Spalt) zwischen Sensorfeld und Membranunterseite gepumpt bzw. abgezogen und getauscht werden kann, sowie Eichlösungen zur automatisierten Kalibrierung verwendet werden können.
Die erfindungsgemäße Lösung besteht in der Trennung von Zellkulturraum und Sensorfeld durch eine biokompatible poröse Membran. So kann die Sensorik effektiv miniaturisiert und optimiert werden, die Zellen wachsen auf einem geeigneten Untergrund und die Messung an den am meisten nährstofflimitierten unteren Zellschichten ist gewährleistet.

Es liegt eine Anordnung vor, in der der Boden eines Wells für die Zellkultur von einer biokompatiblen, porösen Membran gebildet und an den unten ein mittels Planartechnologien erzeugtes auf elektrochemischer Grundlage funktionierendes Sensorarray mit nachgeschalteter Elektronik ankoppelbar ist.
Für einen Einsatz in der Zellkultur ist das Sensorfeld auf der Fläche eines Wells von ggf. mehreren Well-Schalen untergebracht. Dazu sind erhebliche Miniaturisierungen der sensorischen, elektronischen und fluidischen Strukturen notwendig. Die Sensoren erfassen die relevanten Messparameter gleichzeitig, eine gegenseitige Beeinflussung ist durch eine vollständige galvanische Trennung ausgeschlossen.

Die in einen entsprechenden Kunststoff eingefasste Membran bildet das Zellkultursystem und ist über ein Kopplungssystem mit der Sensoreinheit verbunden, so dass durch Abkoppeln ein Nachkalibrieren möglich ist.

Die Anordnung mit Probenraum besteht bspw. aus Polymeren, Polymerblends und Copolymeren, Keramik, Metallen und Legierungen, Glas sowie Verbundwerkstoffen.
Die Membran der vorliegenden Anordnung ist als poröse Membran, bspw. bestehend aus Keramik, Polymeren, Polymerblends, Copolymeren, Metallen, Legierungen, Glas und /oder Verbundwerkstoffen ausgeführt.

Vorteilhaft ist die Membran aus Keramik in homogener Form oder einem aus Schichten bestehendem Aufbau gestaltet. Dabei kann sie entweder biokompatibel oder bioinert sein.

Besonders vorteilhaft ist die Membran aus Hydroxylapatit oder Schichtkeramik mit einer Oberfläche aus Hydroxylapatit, die eine osteoinduktive Unterlage für die Zellkultur bildet, ausgeführt.

Die Membran kann in der Art und Weise funktionalisierend modifiziert sein, dass eine Zelladhäsion, das Zellwachstum oder sonstiges Zellverhalten beeinflusst werden kann.

Die Porengröße beträgt vorteilhaft weniger als 0,3 *µ*m, so dass eine sterile Trennung zwischen Probenraum und Außenseite gewährleistet ist. Besonders vorteilhaft ist die Membran mit einer sehr engen, monomodalen Porenradienverteilung gestaltet, so dass eine scharfe Trenngrenze und zeitlich eng verteiltes Diffusionsverhalten der Analyte gewährleistet ist.

Die vorliegende Erfindung ermöglicht die Durchführung eines Verfahrens zur Messung an Zell- und Gewebekulturen, in dem die zu messenden Proben in einem durch eine poröse Membran von der Messvorrichtung abgetrennten Raum vorliegen, so dass die zu messenden Analyte durch die Membran diffundieren, die Proben nicht in Wechselwirkung mit den Materialien der Messvorrichtung stehen und die Messvorrichtung vor schädigenden oder störenden Einflüssen der Proben geschützt ist.
Bei diesem Verfahren kann die Anordnung einen oder mehrere Sensoren mit Ableitelektronik aufweisen und der Probenraum sowie die Membran kann von der Messvorrichtung getrennt werden, so dass eine Nachkalibrierung oder ein Austausch der Messvorrichtung ermöglicht wird.
Dabei schließt die Membran den Probenraum so ab, dass dieser nach der Trennung von der Messvorrichtung ein steriles und abgeschlossenes System bildet.
Durch den erfindungsgemäßen Einsatz der biokompatiblen Membran können für die Zellen geeignete Oberflächen bereit gestellt werden, die im Falle des Hydroxylapatit sogar eine ideale Grundlage für Knochen-Knorpel-Kulturen darstellen.
Die Anordnung ist dabei als Zellkulturgefäß für die Zell- und Gewebekultur ausgeführt. Hierbei bildet die Membran den Boden des Gefäßes, so dass vermittels des Verfahrens u.a. Messungen an den unteren, vom Medium am wenigsten versorgten Lagen möglich sind.

Vorteilhaft bei der erfindungsgemäßen Anordnung ist, dass der Probenraum und die Membran getrennt oder zusammen sterilisiert werden können (bspw. durch Autoklavieren).

Das Kopplungssystem der erfindungsgemäßen Anordnung ermöglicht die Trennung von Messvorrichtung und Probenraum mit Membran, so dass ein mehrmaliges An- und Abkoppeln ermöglicht ist, und ein definierter Abstandsspalt zwischen Sensoren und Membranunterseite generierbar ist.

Im Rahmen der Erfindung liegt auch, dass die Anordnung als Messkopf ausgeführt ist, der nacheinander an die Membranen einer Probeneinheit mit mehreren einzelnen Probenraum-Membran-Einheiten ankoppelbar ist, so dass Proben automatisiert nacheinander abtastbar sind.

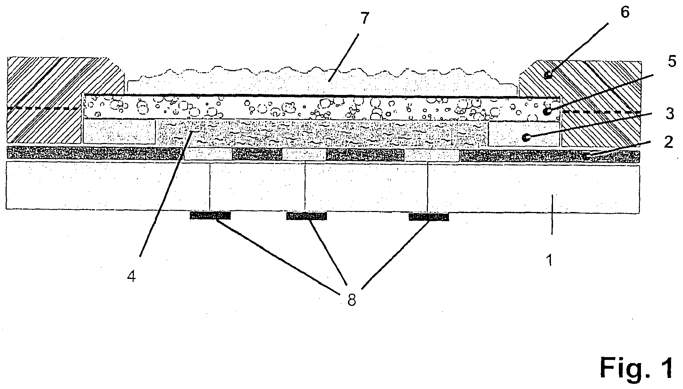

Die Erfindung wird nachfolgend an Hand von Ausführungsbeispielen näher erläutert.
Das grundlegende Funktionsprinzip ist dabei in Fig. 1 dargestellt.

### Ausführungsbeispiel

Auf einem Substrat 1 sind planare elektrochemische Sensoren 2 ausgebildet, die in Dickfilm- und Dispensertechnik in der Weise miniaturisiert sind, dass jeweils ein Sauerstoff-, pH- und Glucose-Sensor auf einer Fläche von ca. 1,75 cm² untergebracht und mit sensornahen Betriebsschaltungen 8 verbunden sind.

Der Zellkulturraum 7, in dem die Zellkultivierung stattfindet, ist von den planaren dickschichttechnologisch erzeugten Sensoren 2 durch eine definiert permeable biokompatible Membran 5 mit einer Dicke von 0,5 bis 2 mm aus Hydroxylapatit mit einer an ihrer Unterseite absolut planen Oberfläche getrennt, wobei die mittlere Porengrößenverteilung 100 bis 200 nm und die Porosität 20 bis 60 % betragen. Der maximale Porendurchmesser beläuft sich auf 300 nm, wodurch eine Sterilbarriere zum Zellkulturraum 7 (Probenraum) hin generiert wird. Zugleich bietet ein geeignetes Transfermedium 4 die Möglichkeit, dass die zu messenden Spezies mit den Sensoren 2 wechselwirken können.

Der gesamte Zellkulturraum ist gegenüber der Substratplatte 1 (Sensorplatte) abnehmbar und dabei seitlich flüssigkeitsdichtend abgeschlossen, so dass ein gemeinsamer Raum 4 (Spalt) ausgebildet ist, der vermittels mikrofluidischer Systeme mit Transfermedium befüllbar ist. Der Raum 4 ist dabei gegenüber der Membran mit Dichtungsringen abgedichtet.

Definierte Randbedingungen liefert ein Spacer 3 zwischen der Substratplatte 1 (Sensorplatte, bspw. in form eines Sensorarrays) und der Membran 5, welcher zum einen die mechanische Stabilität der Membran 5 und zum anderen einen konstant dicken Elektrolytfilm gewährleistet.

Durch einen entsprechenden Verschluss am Rand ist das erfindungsgemäße System durch die Möglichkeit einer Ankopplung, welche einen Verbindungsmechanismus 6 sicherstellt, nachkalibrierbar und die Dauer der Kultivierung in diesem Punkt ist nicht mehr begrenzt.

Im Gegensatz zum Stand der Technik ermöglicht es die erfindungsgemäße Lösung, dass Zellen auf einem optimalen Untergrund kultiviert und dennoch mit präziser Sensorelektronik störungsfrei wichtige Stoffkonzentrationen gemessen werden können. In dreidimensionalen Konstrukten kann nicht-invasiv und zuverlässig in den unteren Schichten gemessen werden. Für die Sensorik ist durch den Ankopplungsmechanismus eine Kalibrationsmöglichkeit vorhanden. Außerdem sind die Sensorsignale nicht durch zum Teil mit biologischen Zellen belegte Sensoren und damit verbundener Diffusionshemmung verfälscht. Zudem kann für die Zellen ein optimaler, biokompatibler Untergrund auch für das adhärente Wachstum zur Verfügung gestellt werden. Hier kann sogar eine funktionalisierende biologische Beschichtung angewendet werden.

Die erfindungsgemäße Anordnung gemäß Fig. 1 kann mit einer Vielzahl zu einem Array angeordneter Sensoren realisiert sein (bspw. mit verschiedenen Sensoren, deren spezifische Analyte gleichzeitig erfasst werden können). Dabei sind planare Sensoren, miniaturisiert nebeneinander angeordnet und sind so ausgeführt, dass keine gegenseitige Beeinflussung stattfindet. Die unmittelbar mit den Sensoren in Kontakt stehende primäre Ableitelektronik ist dabei so realisiert, dass eine galvanische Trennung der Kontakte gewährleistet ist. Mehrere solcher Messzellen können zusammen so nebeneinander angeordnet sein, dass mehrere Proben gleichzeitig, jedoch unabhängig voneinander gemessen werden können.
Die Membran(en) 5 kann/können durch Oberflächenmodifikation an die Anforderungen der Zellkultur angepasst sein. Die kann eine Strukturierung, eine chemische Modifikation zum Einstellen der hydrophilen/hydrophoben Eigenschaft, eine Kopplung bioaktiver Moleküle oder eine sonstige Oberflächenmodifikation sein, wesentlich dabei ist nur, dass die Messung durch die Membran(en) 5 nicht gestört wird.
Der Verbindungsmechanismus 6 (das Kopplungssystem) ist im Beispiel durch einen seitlich abgedichteten Raum verwirklicht, der das Heben und Senken des Zellkulturraumes 7 mit der Membran 5 gegenüber den Sensoren 2 ermöglicht. So kann der Raum 4 (Spalt) erweitert und als Ganzes mit Flüssigkeit gespült werden. Nach dem Absenken umschließen nicht in der Fig. 1 dargestellte Dichtringe unter der Membran 5 einen isolierten Spalt mit den Sensoren 3, so dass die Messung beginnen kann. Ein Vorteil dieser Ausführung ist es, dass besonders gut luftblasenfrei gearbeitet werden kann.

In einer anderen Ausführungsform wird die erfindungsgemäße Anordnung in Gefäße so eingebaut, dass eine kontinuierliche Messung am Gefäßinhalt möglich ist. In einer speziellen Variante handelt es sich bei den Gefäßen um Zell- oder Gewebekulturschalen. Dabei kann das Instrument sowohl im Mediumraum, wie z.B. in der Wand von Bioreaktoren für die Suspensionkultur, als auch direkt unter adhärent wachsenden Zellen eingebaut werden. Diese Einheit kann als Bausatz in verschiedenste Anwendungsbereiche integriert werden.
In einer weiteren Ausführungsform wird die Anordnung in Form eine Multiwell-Platte realisiert, wie sie z.B. für die Zellkultur verwendet wird. Dabei bildet die Membran 5 den Boden je eines Wells, und unter jedem Well wird ein Sensorarray mit Mikrofluidik realisiert, so dass eine simultane Messung unter jedem Well möglich ist. Das Substrat 1 (Platte) ist dabei entweder fest verbunden oder austauschbar, so dass durch An- und Abkoppeln mehrere Platten hintereinander gemessen werden können. In einer Variante wird diese Platte in ein Gerät mit kompletter Messeinrichtung inklusive Steuerungseinrichtung für Temperatur, Gaszusammensetzung und Luftfeuchte integriert, so dass die Proben unter kontinuierlicher Messung inkubiert werden können. In einer weiteren Variante wird das Instrument kompakt und so konstruiert, dass die gesamte Messeinheit in einem Zellkulturinkubator untergebracht werden kann.

In einer weiteren Ausführungsform ist die Anordnung in der Art gestaltet, dass ein Messkopf mit einem Sensorarray und Microfluidik mittels einer Robotik mehrere Messstellen in einer Messplatte mit mehreren Probenräumen, die unten von der Membran und dem Kopplungssystem begrenzt sind, durch wiederholtes An- und Abkoppeln anfährt und so die Messungen der Reihe nach durchführt. Der Vorteil hierbei ist, dass bei gleicher Sensorfläche bzw. gleichem Messspalt mehr Raum für die Ableitelektronik und die Mikrofluidik zur Verfügung steht.

In einer weiteren Ausführungsform wird die erfindungsgemäße Anordnung in einen Bioreaktor integriert, entweder zur Kontrolle von Suspensionkulturen, oder von Kulturen in für die Gewebezüchtung adaptierten Reaktoren, z.B. Durchfluss oder Druck-Perfusionsreaktoren. Mit der erfindungsgemäßen Anordnung können Untersuchungen in der Zellphysiologie durchgeführt werden, die mit bisherigen Anordnungen und Untersuchungsmethoden nicht zugänglich waren.

So können Messungen mit einem oder mehreren Sensoren in einem Sensorarray gleichzeitig auch an solchen Proben durchgeführt werden, bei denen es wichtig ist, den direkten Kontakt zwischen Sensor und Probe zu vermeiden. Dabei ist es von Vorteil, wenn Probenraum und Sensoreinheit durch ein Kopplungssystem getrennt werden können, um
i) den Tausch der Sensoreinheit bzw. des Probenraumes,
ii) die Nachkalibrierung
iii) die Reinigung der Messeinheit zu ermöglichen.

Die erfindungsgemäße Anordnung ermöglicht über einen längeren Zeitraum eine effektive Messung an Zell- und Gewebekulturen, bspw. in einem Array verschiedener Analyte (wie z.B. pH, Glucose, Sauerstoff). So ist es bspw. möglich, die Sensorik der genannten Anordnung unter eine Standard-Zellkultureinheit (15 mm Ø Well einer Kulturschale) einzupassen, damit mehrere Parameter gleichzeitig analysieren zu können und dies dennoch in wirtschaftlicher Weise herzustellen.

Der Vorteil der erfindungsgemäße Anordnung besteht darin, dass Gewebekulturen über längere Zeiträume gemessen werden können, wobei die Probenraumoberfläche steril und biokompatibel ist und die Messwerte unterhalb einer Gewebs- oder Zellschicht gemessen werden können, in dem die Messung der zu bestimmenden Parameter durch die Auflagefläche der erfindungsgemäßen Anordnung von unten möglich ist, so dass Fragestellungen der Zellphysiologie bearbeitet werden können, die mit bisherigen Untersuchungsmethoden gemäß dem Stand der Technik nicht untersucht werden konnten.
Im besonderen Fall der Kultur von Knochen- und / oder Knorpelzellen besteht der Vorteil der erfindungsgemäßen Anordnung darin, dass die Membran aus Hydroxylapatit für diese Zellen kompatibel und mit diesen kombinierbar ist.

Alle in der Beschreibung, dem nachfolgenden Ausführungsbeispiel und der Zeichnung dargestellten Merkmale können sowohl einzeln als auch in beliebiger Kombination miteinander erfindungswesentlich sein.

### Bezugszeichenliste

- 1: Substrat
- 2: elektrochemische Sensoren
- 3: Spacer
- 4: Raum für Transfermedium
- 5: biokompatible Membran
- 6: Verbindungsmechanismus
- 7: Zellkulturraum
- 8: Betriebsschaltungen

## Patentansprüche

1. Anordnung zur Online-Messung löslicher Analyte oder gelöster Gase umfassend ein Substrat (1), mindestens einen Sensor (2), einen Raum (4) und eine Membran (5), wobei das Substrat (1) den Sensor (2) bzw. die Sensoren und die Membran (5) in der Art haltert, dass zwischen dem Sensor (2) oder den Sensoren und der Membran (5) ein Raum (4) generiert ist, die Membran (5) biokompatibel oder bioinert und mit einem Zellkulturraum (7) verbunden ist, der Raum (4) mit einem Transfermedium befüllt sowie in fluidische Systeme integriert ist und der Sensor (2) oder die Sensoren Betriebsschaltungen (8) tragen, über die nachschaltbare Elektronik ankoppelbar ist, wobei die Membran (5) in Kunststoff eingefasst ist und ein Zellkultursystem bildet, in dem die Membran (5) über ein Kopplungssystem mit einer Sensoreinheit verbunden ist und ein Spacer (3) zwischen der Substratplatte (1) und der Membran (5) angeordnet ist, **dadurch gekennzeichnet dass** das Kopplungssystem ein Bajonettverschluss ist, so dass durch mehrmaliges An- und Abkoppeln ein Nachkalibrieren möglich ist, und der Spacer (3) zum einen die mechanische Stabilität der Membran (5) und zum anderen einen konstant dicken Elektrolytfilm gewährleistet, wobei ein definierter Abstandsspalt zwischen Sensoren und Membranunterseite generiert ist.

2. Anordnung gemäß Anspruch 1, **dadurch gekennzeichnet, dass** das Kopplungssystem die Trennung von Messeinrichtung und Probenraum / Zellkulturraum (7) mit Membran (5) ermöglicht, so dass ein mehrmaliges An- und Abkoppeln ermöglicht ist.

3. Anordnung gemäß Anspruch 1 oder 2, **dadurch gekennzeichnet, dass** der Sensor (2) oder die Sensoren elektrochemische Sensoren sind.

4. Anordnung gemäß Anspruch 1 oder 2, **dadurch gekennzeichnet, dass** die Membran (5) porös ist und aus Keramik, Polymeren, Polymerblends, Copolymeren, Metallen, Legierungen, Glas oder / und Verbundwerkstoffen besteht.

5. Anordnung gemäß Anspruch 1 oder 2, **dadurch gekennzeichnet, dass** die Membran (5) eine sehr enge, monomodale Porenradienverteilung besitzt.

6. Anordnung gemäß Anspruch 1 oder 2, **dadurch gekennzeichnet, dass** die Membran (5) eine Dicke von 0,5 bis 2 mm besitzt.

7. Anordnung gemäß Anspruch 1 oder 2, **dadurch gekennzeichnet, dass** die Membran (5) aus einem mehrschichtigen keramischen System besteht, bei dem die obere, mit Zellen in Kontakt bringbare Schicht aus einer Schichtkeramik mit einer Oberfläche aus Hydroxylapatit besteht, wobei alle Schichten die gleichen Eigenschaften bezüglich Größe, Anteil und Verteilung der Poren besitzen.

8. Anordnung gemäß Anspruch 1 oder 2, **dadurch gekennzeichnet, dass** die Membran (5) aus Hydroxylapatit besteht, wobei die mittlere Porengrößenverteilung 100 bis 200 nm, der maximale Porendurchmesser 300 nm und die Porosität 20 bis 60 % betragen.

9. Anordnung gemäß Anspruch 1 oder 2, **dadurch gekennzeichnet, dass** die Sensoren (2) sich nicht gegenseitig physiko-chemisch beeinflussen.

10. Anordnung gemäß Anspruch 1 oder 2, **dadurch gekennzeichnet, dass** der Sensor (2) oder die Sensoren planar ist/sind.

11. Anordnung gemäß Anspruch 1 oder 2, **dadurch gekennzeichnet, dass** die Sensoren (2) ein Sauerstoff-, ein pH- und ein Glucose-Sensor sind, welche auf einer Fläche von ca. 1,75 cm² von dem Substrat gehaltert sind.

12. Anordnung gemäß Anspruch 1 oder 2, **dadurch gekennzeichnet, dass** der Zellkulturraum (7) und Membran (5) getrennt oder zusammen sterilisiert / autoklaviert werden können.

13. Verfahren zur Online-Messung löslicher Analyte oder gelöster Gase unter Verwendung einer Anordnung gemäß einem oder mehrerer der voran stehenden Ansprüche 1 bis 12, bei dem der Probenraum sowie die Membran (5) in zeitlichen Abständen mehrmalig von der Messeinrichtung an- und abgekoppelt werden kann, um ein Nachkalibrieren durch Abkopplung und Koppeln von Eichlösungen, einen Austausch der Sensoren zur Erweiterung der messbaren Parameter und einen Austausch der Probenräume zur Reihenmessung von mehreren Proben vorzunehmen, in dem durch den Bajonettverschluss die Messeinrichtung von dem Probenraum / Zellkulturraum (7) mit Membran (5) getrennt und durch den Spacer (3) ein definierter Abstandsspalt zwischen Sensoren und Membranunterseite generiert wird, wobei die zu messenden Analyte von dem Probenraum / Zellkulturraum (7) durch die Membran (5) diffundieren, der Sensor (2) / die Sensoren physikalisch oder chemische Parameter messen, die von einer nachgeschalteten Elektronik erfasst sowie ausgewertet werden und ein Austausch der Teilanordnung, umfassend Substrat (1), Sensor (2), Raum (4) und Membran (5), ermöglicht wird.

## Claims

1. An arrangement for online measurements of soluble analytes or dissolved gases comprising a substrate (1), at least one sensor (2), a space (4) and a membrane (5), wherein the substrate (1) holds the sensor (2) or the sensors and the membrane (5) in such a way that a space (4) is generated between the sensor (2) or the sensors and the membrane (5), the membrane (5) is biocompatible or bioinert and connected to a cell culture space (7), the space (4) is filled with a transfer medium and integrated in fluidic systems, and the sensor (2) or the sensors carries/carry operating circuits (8) via which downstream electronics can be coupled, wherein the membrane (5) is framed by plastic material and forms a cell culture system in which the membrane (5) is connected to a sensor unit via a coupling system and a spacer (3) is arranged between the substrate plate (1) and the membrane (5), **characterized in that** the coupling system is a bayonet lock so that recalibration is possible by repeated coupling and uncoupling, and the spacer (3) ensures both the mechanical stability of the membrane (5) and a constantly thick electrolyte film, wherein a defined distance space is generated between the sensors and the bottom side of the membrane.

2. The arrangement according to claim 1, **characterized in that** the coupling system allows the separation of the measurement device from the sample area/cell culture space (7) with membrane (5) so that the system can be repeatedly coupled and uncoupled.

3. The arrangement according to claim 1 or 2, wherein the sensor (2) or the sensors are electrochemical sensors.

4. The arrangement according to claim 1 or 2, **characterized in that** the membrane (5) is porous and is made of ceramics, polymers, polymer blends, co-polymers, metals, alloys, glass or/and composite materials.

5. The arrangement according to claim 1 or 2, **characterized in that** the membrane (5) has a very narrow, monomodal pore radius distribution.

6. The arrangement according to claim 1 or 2, **characterized in that** the membrane (5) has a thickness of between 0.5 and 2 mm.

7. The arrangement according to claim 1 or 2, **characterized in that** the membrane (5) consists of a multilayer ceramic system in which the upper layer, which can be brought into contact with cells, is made of layered ceramic material with a surface of hydroxylapatite, wherein all layers show the same properties concerning the size, proportion and distribution of the pores.

8. The arrangement according to claim 1 or 2, **characterized in that** the membrane (5) consists of hydroxylapatite, wherein the mean pore size distribution is between 100 and 200 nm, the maximum pore diameter is 300 nm and the porosity ranges between 20 and 60 %.

9. The arrangement according to claim 1 or 2, **characterized in that** the sensors (2) do not have a physico-chemical influence on one another.

10. The arrangement according to claim 1 or 2, **characterized in that** the sensor (2) or the sensors is/are planar.

11. The arrangement according to claim 1 or 2, **characterized in that** the sensors (2) are an oxygen, a pH and a glucose sensor that are supported by the substrate on an area of about 1.75 cm².

12. The arrangement according to claim 1 or 2, **characterized in that** the cell culture space (7) and the membrane (5) can be sterilized/treated by autoclave separately or together.

13. A method for the online measurement of soluble analytes or dissolved gases by using an arrangement according to one or several of the previous claims 1 to 12, in which the sample area and the membrane (5) can be repeatedly coupled to and uncoupled from the measurement device at time intervals in order to carry out recalibration by coupling and uncoupling calibration solutions, to replace the sensors for expanding the measurable parameters, and to replace the sample areas for the serial measurement of several samples, achieved by the separation of the measurement device from the sample area/cell culture space (7) with membrane (5) by the bayonet lock and by the generation of a defined space between the sensors and the bottom side of the membrane by the spacer, wherein the analytes to be measured diffuse from the sample area/cell culture space (7) through the membrane (5), the sensor (2)/the sensors measure/s physical or chemical parameters, which are recorded and analyzed by downstream electronics, and the replacement of the parts arrangement comprising the substrate (1), the sensor (2), the space (4) and the membrane (5) is made possible.

## Revendications

1. Dispositif pour la mesure en ligne des analytes solubles ou des gaz dissous contenant un substrat (1), au moins un détecteur (2), un espace (4) et une membrane (5) et le substrat (1) supporte le détecteur (2) ou les détecteurs et la membrane (5) d'une telle façon qu'un espace est généré entre le détecteur (2) ou les détecteurs et la membrane (5), que la membrane (5) est biocompatible ou biologiquement inerte et liée avec l'espace de culture cellulaire (7), que l'espace (4) est rempli d'un support de transfert et intégré dans les systèmes fluidiques et que le détecteur (2) ou les détecteurs sont munis des circuits de fonctionnement (8), auxquels l'électronique à monter en aval peut être raccordé, que la membrane (5) est bordée de plastique et forme un système de cultures cellulaires, dans lequel la membrane (5) est liée au dispositif détecteur par un système de couplage et un espaceur (3) est disposé entre la plaque de substrat (1) et la membrane (5), est **caractérisé en ce que** le système de couplage est un verrouillage par baïonnette de sorte qu'un recalibrage devienne possible par la connexion et déconnexion répétées et que l'espaceur (3) assure soit la stabilité mécanique de la membrane (5), soit un film d'électrolyte d'une épaisseur constante et qu'une fente définie est générée entre les détecteurs et la face inférieure de la membrane.

2. Dispositif suivant la revendication 1 est **caractérisé en ce que** le système de couplage permet de séparer le dispositif de mesure et l'espace échantillons / espace de culture cellulaire (7) avec la membrane (5) de sorte qu'une connexion et déconnexion répétées soit possible.

3. Dispositif suivant la revendication 1 ou 2 est **caractérisé en ce que** le capteur (2) ou les capteurs sont des capteurs électrochimiques.

4. Dispositif suivant la revendication 1 ou 2 est **caractérisé en ce que** la membrane (5) est poreuse et se compose de céramique, polymères, mélange de polymères, co-polymères, métaux, alliages, verre et/ou matériaux composites.

5. Dispositif suivant la revendication 1 ou 2 est **caractérisé en ce que** la membrane (5) possède une répartition monomodale, très étroite des rayons de pores.

6. Dispositif suivant la revendication 1 ou 2 est **caractérisé en ce que** la membrane (5) possède une épaisseur allant de 0,5 à 2 mm.

7. Dispositif suivant la revendication 1 ou 2 est **caractérisé en ce que** la membrane (5) se compose d'un système céramique multicouche dont la couche supérieure, qui peut être mise en contact avec les cellules, se compose d'une céramique de stratification avec une surface en hydroxyapatite et que toutes les couches possèdent les mêmes caractéristiques concernant la taille, portion et répartition des pores.

8. Dispositif suivant la revendication 1 ou 2 est **caractérisé en ce que** la membrane (5) se compose de hydroxyapatite et la répartition moyenne de taille de pores est de 100 à 200 nm, le diamètre maximal de pores est de 300 nm et la porosité est de 20 à 60 %.

9. Dispositif suivant la revendication 1 ou 2 est **caractérisé en ce que** les détecteurs (2) ne s'influencent pas réciproquement de façon physicochimique.

10. Dispositif suivant la revendication 1 ou 2 est **caractérisé en ce que** le capteur (2) ou les capteurs sont planes.

11. Dispositif suivant la revendication 1 ou 2 est **caractérisé en ce que** les capteurs (2) sont un détecteur d'oxygène, de pH et de glucose qui sont fixés sur une surface d'env. 1,75 cm² du support.

12. Dispositif suivant la revendication 1 ou 2 est **caractérisé en ce que** l'espace de culture cellulaire (7) et la membrane (5) peuvent être stérilisés / autoclavés séparément ou conjointement.

13. Dispositif pour la mesure en ligne des analytes solubles ou des gaz dissous en utilisant un dispositif suivant une ou plusieurs des revendications précédentes 1 à 12, avec lequel l'espace échantillons et la membrane (5) peuvent être connectés et déconnectés quelques fois du dispositif de mesure à intervalles de temps pour permettre le recalibrage par déconnexion et connexion des solutions de calibrage, l'échange des détecteurs pour étendre les paramètres mesurables et l'échange des espaces échantillons pour mesurer plusieurs échantillons en série en séparant le dispositif de mesure de l'espace échantillons / espace de culture cellulaire (7) avec la membrane (5) par le verrouillage par baïonnette et en générant une fente définie entre les détecteurs et la face inférieure de la membrane par l'espaceur (3), et les analytes à mesurer diffusent de l'espace échantillons / espace de culture cellulaire (7) à travers de la membrane (5), le détecteur (2) / les détecteurs mesurent des paramètres physiques ou chimiques, qui sont enregistrés et évalués par une électronique mise en aval, et une échange de l'ensemble contenant le substrat (1), détecteur (2), espace (4) et la membrane (5) devient possible.
